(19) Europäisches Patentamt
European Patent Office
Office européen des brevets

(11) **EP 4 183 870 A1**

(12) **EUROPEAN PATENT APPLICATION**
published in accordance with Art. 153(4) EPC

(43) Date of publication:
**24.05.2023 Bulletin 2023/21**

(21) Application number: **21843270.6**

(22) Date of filing: **15.07.2021**

(51) International Patent Classification (IPC):
**C12N 5/0775** $^{(2010.01)}$

(52) Cooperative Patent Classification (CPC):
**C12N 5/06**

(86) International application number:
**PCT/CN2021/106399**

(87) International publication number:
**WO 2022/012608 (20.01.2022 Gazette 2022/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.07.2020 CN 202010691416**

(71) Applicant: **Shanghai Wolwo Stem Cell Technology Co., Ltd.**
**Shanghai 200233 (CN)**

(72) Inventor: **HU, Gengxi**
**Shanghai 200233 (CN)**

(74) Representative: **Pace Napoleone, Maria et al**
**De Simone & Partners S.r.l.**
**Via Vincenzo Bellini, 20**
**00198 Roma (IT)**

Remarks:
The complete document including Reference Table(s) and the Sequence Listing(s) can be downloaded from the EPO website

(54) **STEM CELL CULTURING METHOD**

(57) Provided is a method of culturing stem cells, comprising a step of culturing stem cells in an atmospheric environment comprising an additional pressure applied in addition to an atmospheric pressure, wherein the additional pressure may be about 60 - 140 mmHg. Also provided are stem cells obtained according to the method of culturing.

EP 4 183 870 A1

**Description**

**FIELD OF THE INVENTION**

This application pertains to the field of stem cell technology, and specifically a method of culturing stem cells.

**BACKGROUND**

Stem cells are multipotential cells capable of self-replication, which can differentiate into various functional cells under appropriate conditions. Embryonic stem cells are totipopential, which has the highest differentiation potential. However, their clinic application is still plagued by uncertainty due to some concerns like tumorigencity and morality. Mesenchymal stem cells (MSCs), as a member of the family of stem cells, are present in a number of tissues (e.g., bone marrow, umbilical cord blood and umbilical cord tissue, placenta tissue, adipose tissue, etc.) and have multiple potencies of differentiation. This type of stem cells are capable of differentiating into various mesenchymal cells (e.g., osteoblasts, chondroblasts, adipoblasts, etc.) or non-mesenchymal cells and have unique functionalities of cytokine secretion, immunomodulation and anti-inflammation, which have been reported as useful in treating immune-associated diseases such as systemic lupus erythematosus, Crohn's disease and graft-versus-host disease. Mesenchymal stem cells have also been reported as can ameliorate atherosclerosis in some basic researches and clinical trials.

High efficiency in culturing of stem cells is desired, for which a conventional solution is screening for an optimal culture medium. There have rarely been reported studies on impacts of other factors on culturing efficiency and activity of stem cells, such as oxygen concentration and pressure. As reported, the normal oxygen tension in human bone marrow is 3.591 ~ 6.517 kPa, corresponding to an oxygen volume fraction of 4% ~ 7%. Accordingly, mesenchymal stem cells live in a hypoxic environment under normal condition. Meanwhile, it has been reported that additional pressure provides improved culture of mesenchymal stem cells. Nevertheless, there have never been reported any studies and optimization with respect to atmospheric parameters in culturing condition for stem cells, like oxygen concentration and air pressure. There exists the persistent need for improved methods of culturing stem cells in research and industry realms.

**SUMMARY OF THE INVENTION**

The present invention provides a novel improved method of culturing stem cells based on studies and optimization of the atmospheric environment in culturing condition. Stem cells cultured using the method of the present invention exhibit improved biological activities including proliferation capability, independent viability, differentiation potency, sustained sternness and senescence resistance, etc.

In a first aspect, provided is a method of culturing stem cells, comprising a step of culturing stem cells in an atmospheric environment comprising an additional pressure applied in addition to an atmospheric pressure, wherein the additional pressure may be 60 - 140 mmHg. The additional pressure may be static or dynamic, such as a pressure of periodic fluctuation.

In an embodiment, the atmospheric environment comprises an oxygen concentration of 2% - 20%, preferably a hypoxic environment, such as a low oxygen concentration of 2% - 7%.

In the present disclosure, also provided are stem cells obtained via culturing using the method of the present invention.

In the present disclosure, the term "stem cell(s)" does not include cells isolated or obtained from a human embryo that is older than 14 days after fertilization or has undergone *in vivo* development. The stem cells are preferably mesenchymal stem cells.

**BRIEF DESCRIPTION OF THE DRAWINGS**

**Figure 1** shows expansion fold for hair follicle mesenchymal stem cells cultured under different conditions.
**Figure 2** shows passage numbers and expansion fold for hair follicle mesenchymal stem cells cultured under different conditions. Therein, passage numbers are expressed as "Pn", wherein n is a positive integer.
**Figure 3** shows CFU rate for umbilical mesenchymal stem cells and adipose mesenchymal stem cells cultured under different conditions.
**Figure 4** shows osteogenesis rate (figure 4A) and adipogenesis rate (figure 4B) for hair follicle mesenchymal stem cells cultured under different conditions.
**Figure 5** shows an additional pressure fluctuating in a sinusoidal or sinusoid-like manner within the range of 75 - 115 mmHg applied in addition to an atmospheric pressure according to an embodiment of the invention.

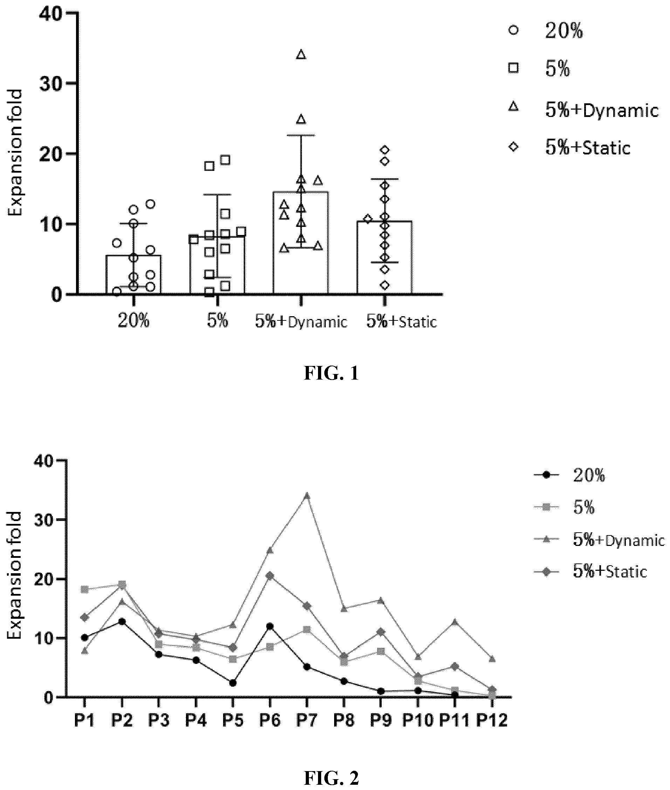

In figures 1 to 5, said different (culturing) conditions distinguish merely in the differential features of atmospheric environment as indicated in the figures and in the followings: "20%" means an oxygen concentration of 20% and no additional pressure; "5%" means an oxygen concentration of 5% and no additional pressure; "5%+Static" means an oxygen concentration of 5% and an additional constant pressure of 95 mmHg applied in addition to an atmospheric pressure; "5%+Dynamic" means an oxygen concentration 5% and an additional pressure applied in addition to an atmospheric pressure, wherein the additional pressure changes in a sinusoidal or sinusoid-like periodic fluctuation within the range of 75 - 115 mmHg at a frequency of 14 times/minute.

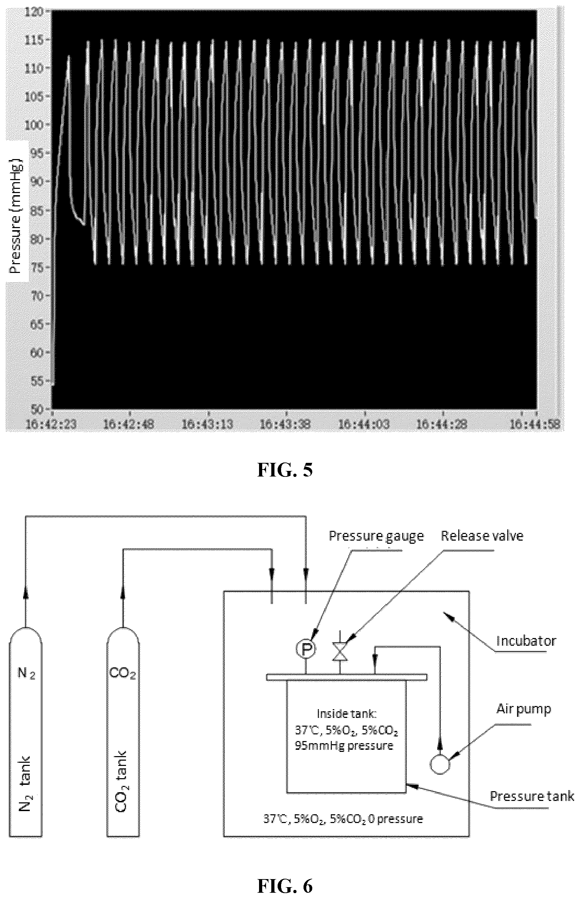

Figure 6 shows schematically a device for hypoxia-and-static pressure (5%+Static), wherein cells are cultured in a pressure tank under an oxygen concentration of 5% and an additional pressure applied in addition to an atmospheric pressure, wherein the additional pressure is maintained at 95 mmHg.

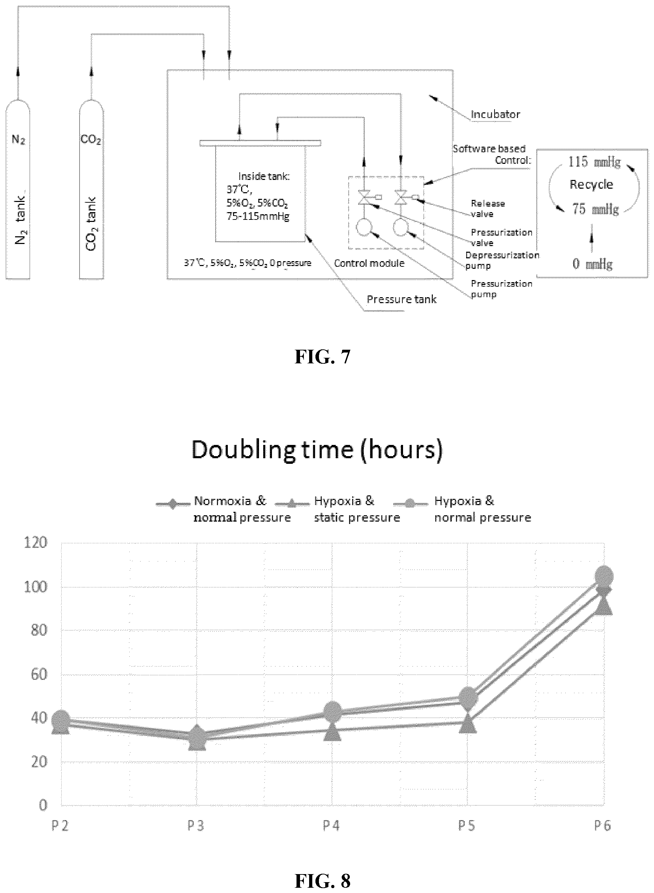

Figure 7 shows schematically a device for hypoxia-and-dynamic pressure (5%+Dynamic), wherein cells are cultured in a pressure tank under an oxygen concentration of 5% and an additional pressure applied in addition to an atmospheric pressure, wherein the additional pressure changes in a sinusoidal or sinusoid-like periodic fluctuation within the range of 75 - 115 mmHg. In the figure, the arrowed line from the control module to the pressure tank represents the gas circuit for pressurization, and the arrowed line from the pressure tank to the control module represents the gas circuit for depressurization; and the "control module" comprises software-based control and a pressurization air pump, depressurization air pump, a release valve and a pressurization valve in the gas circuits.

Figure 8 shows doubling time for the hair follicle mesenchymal stem cells cultured under different conditions in Example 5.

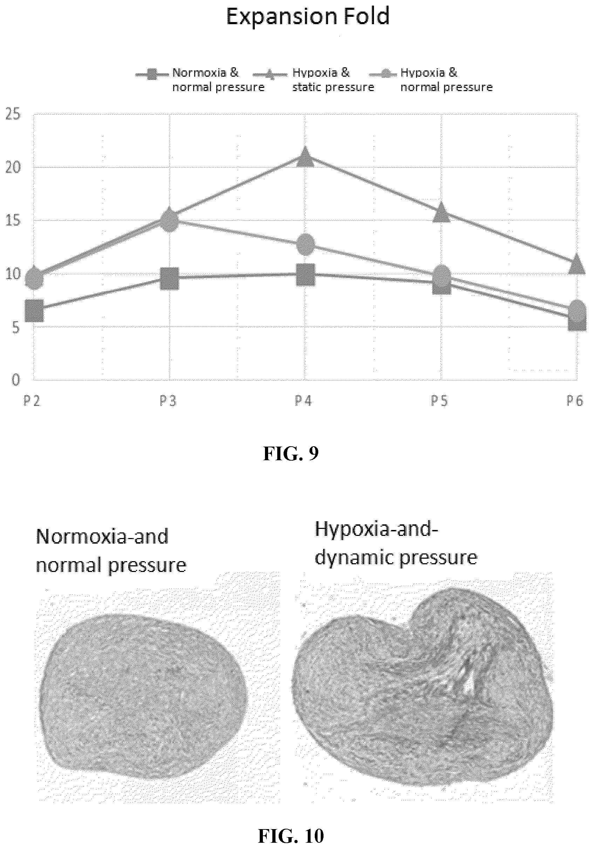

Figure 9 shows expansion fold for the hair follicle mesenchymal stem cells cultured under different conditions in Example 5.

In figures 8 and 9, said different conditions distinguish merely in the differential features of atmospheric environment as indicated in the figures and in the followings: "normoxia-and-normal pressure" means an oxygen concentration of 20% and no additional pressure; "hypoxia-and-normal pressure" means an oxygen concentration of 3% and no additional pressure; "hypoxia-and-static pressure" means an oxygen concentration of 3% and an additional constant pressure of 60 mmHg applied in addition to an atmospheric pressure.

Figure 10 shows a comparison of chondrogenic differentiation between the hair follicle mesenchymal stem cells cultured under different conditions in Example 6. Therein, said different conditions distinguish merely in the differential features of atmospheric environment as indicated in the figure and in the followings: "normoxia-and-normal pressure" means an oxygen concentration of 20% and no additional pressure; "hypoxia-and-dynamic pressure" means an oxygen concentration of 3% and an additional pressure applied in addition to an atmospheric pressure, wherein the additional pressure changes in a sinusoidal or sinusoid-like periodic fluctuation within the range of 60 - 135 mmHg at a frequency of 60 times/minute.

## DETAILED DESCRIPTION OF THE INVENTION

As used herein, the articles "a" and "an" mean "at least one" and thus encompass plurality, unless otherwise specified.

In the present disclosure, for the described technical features such as components, amounts, steps, condition parameters, parameter values, elements, connection relationship and working relationship, arrangements of combination are not limited to the embodiments and examples described herein and are all encompassed in the scope of the present invention, unless otherwise specified.

In the present disclosure, unless otherwise specified, a value range defined by two ends represents specific disclosure of each real number within the range and all ranges defined by any two of them. And, when a parameter is assigned with a multiplicity of alternative values or value ranges, ranges defined by any two of the values and ends of the ranges are encompassed in the scope of the present invention as long as the ranges do not exceed the broadest range as specified, unless otherwise specified. For instance, in an embodiment, when the frequency of periodic fluctuation is described to be 12 - 100 times/minute, such as 13 - 18 times/minute, 40 - 80 times/minute, 13 - 15 times/minute or 50 - 70 times/minute, it can be understood as describing that besides these specified ranges, the frequency of periodic fluctuation can further be, for example, 13 - 80 times/minute, 15 - 70 times/minute, 18 - 50 times/minute, etc., all falling within the range of 12 - 100 times/minute.

In the present disclosure, unless otherwise specified, a value with or without being preceded by the term "about", "around" or "approximately" covers equivalents within a reasonable range of approximation of $\pm 10\%$, $\pm 5\%$, $\pm 3\%$, $\pm 2\%$, $\pm 1\%$ or $\pm 0.5\%$ around the specified value.

In the present disclosure, unless defined otherwise, all technical and scientific terms used herein have the same meanings as commonly understood by one of ordinary skills in the fields to which this invention pertains, especially the fields of stem cells and mesenchymal stem cells, such as those definitions in text books, laboratory manuals and science and technical references.

Provided herein is a method of culturing stem cells, comprising a step of culturing stem cells in an atmospheric environment comprising an additional pressure applied in addition to an atmospheric pressure, wherein the additional pressure may be about 60 - 140 mmHg.

In the present disclosure, the term "atmospheric environment" refers to the air condition in the environment where the cell culture locates, such as the atmospheric environment in an incubator, which can be mainly characterized by the air composition and pressure. Normally, for cell culturing, including stem cell culture, the atmospheric environment comprises an atmospheric pressure, about 5% $CO_2$ and a relative humidity of about 95%.

The method of the present invention includes applying an additional pressure of about 60 - 140 mmHg in addition to an atmospheric pressure. As understood, the normal diastolic pressure in human is 60 - 90 mmHg and the normal systolic pressure 140 - 90 mmHg. Accordingly, with the additional pressure, the present invention advantageously mimics the pressure environment the stem cells are subjected to *in vivo* in human. In some embodiments, the additional pressure may be about 60 - 130 mmHg, about 70 - 120 mmHg, about 75 - 115 mmHg, about 80 - 110 mmHg, about 85 - 100 mmHg or about 90 - 95 mmHg. For instance, the additional pressure and control may be implemented using the gas pipeline system and the pressure sensing device of incubators.

In some embodiments, "an atmospheric pressure" refers to the environmental atmospheric pressure without additional pressure, i.e., the normal pressure. Preferably, "an atmospheric pressure" refers to one standard atmospheric pressure, i.e., 760 mmHg.

In the present invention, the additional pressure may be static (i.e., constant) or dynamic. In some embodiments, the additional pressure is static, i.e., constant, such as being constant at a value within the range of about 60 - 130 mmHg, about 70 - 120 mmHg, about 75 - 115 mmHg, about 80 - 110 mmHg, about 85 - 100 mmHg or about 90 - 95 mmHg, such as at about 95 mmHg. In the present disclosure, a pressure being "constant" includes the situation wherein the pressure is substantially constant, which means allowing a fluctuation of an amplitude not more than 10% and preferably not more than 5%, 3%, 2%, 1% or 0.5% relative to the nominal pressure value, with infinitesimal fluctuation approaching 0% including zero fluctuation being preferred. Persons in the art know the means for maintaining a stable atmospheric environment (including a constant air pressure) for cell culturing, such as conventional incubators and the gas pipeline system and the monitoring system therein. For instance, in an embodiment, a pressure tank is placed in an incubator and the cells are cultured in the tank; the tank is pneumatically pressurized via pumping gas inside; when the pressure reaches a set value, without stopping the pneumatic pressurization, the release valve is slowly opened such that the incoming gas and the outgoing gas reach a dynamic balance, whereby the atmosphere in the tank is maintained at the set value.

In some embodiments, the additional pressure is dynamic, which is referred to as a "dynamic pressure" herein below. In some embodiments, the additional pressure changes by way of periodic fluctuation within the range of 60 - 140 mmHg. The periodic fluctuation may be of a fixed frequency or a variable frequency, and the amplitudes of individual periods may be identical or different. Accordingly, taking the range of 60 - 140 mmHg for example, the two ends of the range refer to the lowest trough and the highest crest. Preferably, the fluctuation range (i.e., span) of the additional pressure is about 70 - 120 mmHg and more preferably about 75 - 115 mmHg, wherein 75 mmHg is the median of normal diastolic pressures (60 - 90 mmHg) and 115 mmHg is the median of normal systolic pressures (140 - 90 mmHg) in human. Accordingly, the amplitude of the additional dynamic pressure according to the invention advantageously mimics the periodically changing pressure environment the stem cells are subjected to *in vivo* in human. Preferably, the fluctuation is a sinusoidal or sinusoid-like periodic fluctuation, as illustratively shown in figure 5.

For instance, the dynamic pressure may be applied by means of the method as follows: connecting the chamber wherein the culture locates and a pressure modulation device in gas communication and pressurizing the chamber by the pressure modulation device. In an embodiment, the pressure modulation device comprises a pressurization gas circuit and a depressurization gas circuit, wherein the chamber where the culture locates is communicated with the pressurization gas circuit and the depressurization gas circuit, and the pressurization gas circuit is connected to the gas source. The chamber may be the inside space of a culturing device such as an incubator, a pressure tank or pressure chamber and a culture tank, where the culture locates. The gas source may be, for example, a gas storage tank or the environmental atmosphere surrounding the culture chamber. The gas source provides air of the desired composition according to the present invention. The pressurization gas circuit comprises a pressurization air pump and optionally a valve such as a pressurization valve. The depressurization gas circuit comprises a valve such as a release valve and optionally a depressurization air pump. In an embodiment, under the software-based control, gas from the gas source is delivered into the chamber where the culture locates by the pressurization air pump through the pressurization gas circuit. When the air pressure reaches the set upper-limit upon detection, the pressurization gas circuit is closed and meanwhile the release valve is opened to decrease the air pressure in the chamber where the culture locates through the depressurization gas circuit; when the air pressure decreases to the set lower-limit, depressurization is ceased and the depressurization gas circuit closed, while the pressurization gas circuit is reopened; and the above is repeated in cycles to form the air pressure fluctuation. The air pumps and valves may communicate with the software-based control to implement a programmed operation and control of the pressurization and depressurization, so that the pressure in the pressure tank changes in a periodic fluctuation over time, such as a sinusoidal or sinusoid-like fluctuation. In an embodiment, the depressurization gas circuit comprises a depressurization pump, which pumps air out of the chamber to decrease the pressure therein. In another embodiment, the depressurization gas circuit may omit the depressurization air pump for pumping air out of the culture chamber, while implementing depressurization by releasing the pressure in the chamber through the release valve, taking advantage of the differential pressure between the outside and the inside of the chamber.

In another embodiment, the pressure modulation device comprises a pneumatic cylinder, a piston and a piston cylinder. The chamber where the culture locates is communicated with the piston cylinder and the piston is driven by the pneumatic cylinder to move in the piston cylinder to thereby change the air pressure in the chamber. The reciprocating movement of the piston in the cylinder causes periodic change of the gas volume in the chamber and thereby change of air pressure in periodic pulses, wherein the change of air pressure may be designed and controlled as desired, such as a sinusoidal or sinusoid-like change.

In some embodiments, the additional dynamic pressure changes by way of periodic fluctuation at a frequency of about 12 - 100 times/minute, preferably about 13 - 18 times/minute, about 40 - 80 times/minute, about 13 - 15 times/minute or about 50 - 70 times/minute, such as about 12, 13, 14, 15, 16 or 60 times/minute. In some embodiments, the frequency of the periodic fluctuation of the additional dynamic pressure is constant. As understood, the normal respiratory rate in human is 12 - 20 times/minute and the heart rate in healthy adults is 60 - 100 times/minute. Accordingly, the frequency of the additional dynamic pressure according to the invention advantageously mimics the periodically changing pressure environment the stem cells are subjected to *in vivo* in human.

In the present invention, for the air composition in the atmospheric environment, the other parameters than oxygen concentration may be designed by referring to or following the conventional settings for stem cell culturing, which may comprises, for example, a $CO_2$ level of about 5% and a relative humidity of about 95%. In some embodiments, the atmospheric environment comprises about 2% - 20% of oxygen and the rest selected from the group consisting of the other components in air than oxygen, any other gases of no harm to stem cells, or combinations thereof, such as $N_2$, $CO_2$ or a combination thereof. In some embodiments, the atmospheric environment comprises an oxygen concentration of about 2% - 8%, about 2% - 7%, about 2% - 5%, about 3% - 7%, about 4% - 7%, about 5% - 7%, such as about 3%, about 5%. Persons in the art know the means for maintaining a stable atmospheric environment (including a stable air composition) for cell culturing, such as conventional incubators and the $CO_2$ control system and the humidity control system therein. For instance, a three-gas incubator may be used to generate a low oxygen concentration.

The method of the present invention can be used to culture various stem cells including embryonic stem cells, adult stem cells and induced pluripotent stem cells. The adult stem cells may be, for example, adipose, endometrial, hair follicle or umbilical stem cells. Preferably, the stem cells are human stem cells. In the present invention, the term "stem cell(s)" does not include cells isolated or obtained from a human embryo that is older than 14 days after fertilization or has undergone *in vivo* development. In some embodiments, the method of the present invention is used for culturing mesenchymal stem cells such as (human) adipose, endometrial, hair follicle or umbilical mesenchymal stem cells.

In some embodiments, culturing stem cells under the additional pressure is or comprises amplification, maintenance and/or passaging of primary cells. In some embodiments, culturing stem cells under the additional pressure is or comprises amplification, maintenance and/or passaging offspring cells. In some embodiments, cells are cultured, expanded, maintained and passaged under the additional pressure since the primary generation.

In the present invention, culturing conditions other than atmospheric environment, such as culture medium and temperature, may be designed by referring to or following the conventional settings for stem cell culturing. For example, any medium for culturing stem cells such as mesenchymal stem cells can be used in the method of the present invention. For example, any temperature appropriate for culturing stem cells such as mesenchymal stem cells can be used in the method of the present invention, which may be normally 36.5 - 37.5 °C, preferably 37 °C.

Also provided herein are stem cells obtained via culturing using the method of the present invention. Stem cells cultured using the method of the present invention exhibit improved biological activities, including proliferation capability, independent viability, differentiation potency, sustained stemness and senescence resistance, etc.


## EXAMPLES

The present invention will be further described in details with reference to the examples below. It should be understood that these examples are merely provided for the purpose of illustration, with no intention to limit the scope of the invention in any sense.

In Examples 1, 2, 3 and 4, the term "different conditions" and the term "different culturing conditions" refer to the culturing conditions distinguishing from one another in comparison merely in the unique features of atmospheric environment as indicated below, which are also referred to as "the four conditions" herein blow, wherein the pressures are all expressed as gage pressure, i.e., the additional pressure applied in addition to the atmospheric pressure:

**Normoxia (20%):** an oxygen concentration of 20% and no additional pressure;

**Hypoxia (5%):** an oxygen concentration of 5% and no additional pressure;

**Hypoxia-and-static pressure** (5%+Static): an oxygen concentration of 5% and an additional constant pressure of 95 mmHg applied in addition to an atmospheric pressure: specifically, a pressure tank was placed in a hypoxia incubator (ESCO Company) and the cells were cultured in the tank; the tank was pneumatically pressurized via pumping gas inward; when the pressure reached 95 mmHg, without stopping the pneumatic pressurization, the release valve was slowly opened such that the incoming gas and the outgoing gas reached a dynamic balance, whereby the atmosphere in the tank was maintained at 95 mmHg; details were as shown in figure 6; and

**Hypoxia-and-dynamic pressure (5%+Dynamic):** an oxygen concentration of 5% and an additional pressure applied in addition to an atmospheric pressure, wherein the additional pressure changed in a sinusoidal or sinusoid-like periodic fluctuation within the range of 75 - 115 mmHg at a frequency of 14 times/minute (figure 5). As depicted in figure 7, a pressure tank was placed in a hypoxia incubator (ESCO Company) and the cells were cultured in the tank. The pressure tank was communicated with the pressurization gas circuit and the depressurization gas circuit. In the figure, the arrowed line from the control module to the pressure tank represents the pressurization gas circuit, the arrowed line from the pressure tank to the control module represents the depressurization gas circuit, and the "control module" comprises software-based control and a pressurization air pump, depressurization air pump, a release valve and a pressurization valve in the gas circuits. Under the software-based control, the pressurization valve in the pressurization gas circuit was opened, and the pressurization air pump was turned on to pneumatically pressurize the pressure tank by pump the air from the incubator into the pressure tank. When the air pressure reached 115 mmHg upon detection, the pressurization air pump was turned off and the pressurization valve closed in the pressurization gas circuit to stop pressurization, and meanwhile the release valve in the depressurization gas circuit was open and the depressurization air pump was turned on to pump air out of the tank to decrease the pressure therein. When the pressure deceased to 75 mmHg, the release valve was closed and the depressurization air pump turned off in the depressurization gas circuit to stop depressurization, while the pressurization gas circuit was reopened by opening the pressurization valve and turning on the pressurization air pump. The steps were repeated in cycles, and the frequency of alternation was programmedly controlled at 14 times/minute.

In Example 5, the term "different conditions" refers to the culturing conditions distinguishing from one another in comparison merely in the unique features of atmospheric environment as indicated below, which are also referred to as "the three conditions" herein blow, wherein the pressures are all expressed as gage pressure, i.e., the additional pressure applied in addition to the atmospheric pressure:

**Normoxia (20%) and normal pressure:** an oxygen concentration of 20% and no additional pressure;

**Hypoxia (3%) and normal pressure:** an oxygen concentration of 3% and no additional pressure;

**Hypoxia-and-static pressure (3%+Static):** an oxygen concentration of 3% and an additional constant pressure of 60 mmHg applied in addition to an atmospheric pressure: specifically, a pressure tank was placed in a hypoxia incubator (ESCO Company) and the cells were cultured in the tank; the tank was pneumatically pressurized via pumping gas inside; when the pressure reached 60 mmHg, without stopping the pneumatic pressurization, the release valve was slowly opened such that the incoming gas and the outgoing gas reached a dynamic balance, whereby the atmosphere in the tank was maintained at 60 mmHg.

In Example 6, the term "different conditions" refers to the culturing conditions distinguishing from each other in comparison merely in the unique features of atmospheric environment as indicated below, wherein the pressures are all expressed as gage pressure, i.e., the additional pressure applied in addition to the atmospheric pressure:

**Normoxia (20%) and normal pressure:** an oxygen concentration of 20% and no additional pressure;

**Hypoxia-and-dynamic pressure (3%+Dynamic):** an oxygen concentration of 3% and an additional pressure applied in addition to an atmospheric pressure, wherein the additional pressure changed in a sinusoidal or sinusoid-like periodic fluctuation within the range of 60 - 135 mmHg at a frequency of 60 times/minute. The dynamic pressure was applied at the specified pressure range and alternation frequency under the software-based control using the device as depicted in figure 7, as specified above for the hypoxia-and-dynamic pressure conditions in Examples 1 to 4.

**Example 1: Comparison of fold expansion between hair follicle mesenchymal stem cells cultured under different conditions**

1) Intact human hair follicle tissues were placed at the bottom of a 1.5 mL Eppendorf tube using a mocroforcep, into which the enzymolysis solution TrypLE (Gibco - 12604021) was added at 5 $\mu$L per hair follicle, followed by standing in the incubator at 37 °C and 5% $CO_2$ for 3 hours, with gently flicking the tube bottom for mixing every one hour.

2) After 3 hours of enzymolysis, as observed under microscopy, the outer hair root sheath of the hair follicle was completely dissolved while the hair shaft incompletely. The enzymolysate was thoroughly mixed by 10 blowings with a 100 - 1000$\mu$L pipette, without removing the enzymolysis leftover. After standing for 1 minute for the undigested hair shaft to settle to the bottom of the Eppendorf tube, the enzymolysate suspension on top was pipetted as the primary mesenchymal stem cells.

Mesenchymal stem cell suspension pooled from enzymolysis of 5 hair follicles was added into 6-well plates at 25 $\mu$L/well and resuspended by adding 2 mL of the Amniocyte Culture Medium (commercially available from Guangzhou Baiyunshan Baidi Biopharmaceutical Co., Ltd.). The 6-well plates were respectively incubated under the four conditions of normoxia (20%), hypoxia (5%), hypoxia-and-static pressure (5%+Static) and hypoxia-and-dynamic pressure (5%+Dynamic) at 37 °C, with medium refreshing every 3 days.

3) At day 10 to day 12 of the cultivation, when cell density reaching 80% or more in the 6-well plate on observation, the medium was removed and 0.5 mL of TrypLE digestion solution was added to the bottom of the well, followed by digestion at 37 °C for 3 minutes in the incubator. 1 mL of the Amniocyte Culture Medium was added into the 6-well plate to end digestion. The supernatant was collected into a 15 mL centrifuge tube, and the plate was rinsed with 1 mL of the Amniocyte Culture Medium and the rinse was pooled into the tube. After centrifugation at 1500 r.p.m. for 5 minutes, the supernatant was discarded and 1 mL of the Amniocyte Culture Medium added for resuspension. The cells were inoculated into a T25 culture flask at a specified count and then cultured into the hair follicle mesenchymal stem cells of passage P1.

4) The cells were passaged in series to P12 and maintained under the four conditions throughout the process respectively. For each passage, Expansion fold = cell count of the recovery/cell count of the inoculation. In this Example, when the passaging density was accurately 5000 cells/cm$^2$ and the surface area of a T25 culture flask being 25 cm$^2$, each inoculation into a T25 culture flask comprised $1.25\times10^5$ cells and the calculation of expansion fold was "expansion fold = cell count of each recovery / ($1.25\times10^5$)".

As shown by the results in figure 1 and figure 2, the mesenchymal stem cells cultured under hypoxia exhibited increased fold expansion than those cultured under normoxia. Pressurization further enhanced fold expansion. Mesenchymal stem cells under the condition of hypoxia-and-dynamic pressure exhibited the highest expansion fold.

**Example 2: Comparison of colony-formation capacity between mesenchymal stem cells cultured under different conditions**

Colony formation is an effective measurement of cell proliferation capacity. The cell population composed of the offspring of a single cell cultured *ex vivo* is referred to as a clone, where each clone comprises 50 cells or more, sizing between 0.3 - 1.0 mm. The count of colony-formation rate allows for quantitative analysis of proliferation potential and observation of proliferation capacity and independent viability for cells.

**Human umbilical mesenchymal stem cell experiment:**

1) Processing of intact human umbilical tissue: The umbilical cord was cut off 1cm at both ends and rinsed repeatedly to remove blood. The amniotic membrane layer was torn open along the umbilical vein and spread flat using a small surgical scissor and a tissue forceps. The two umbilical arteries and the umbilical vein in the tissue were removed using a tissue forceps.

2) The tissue with blood vessels removed was transferred into 10 ml of digestion enzyme solution TrypLE (Gibco - 12604021) in a 50ml centrifuge tube, wherein the tissue was scissored into pieces of around 1mm$^3$. The tubes were then film-sealed and transferred to digestion on a shaker at 37 °C and a frequency of 60 - 80 times/minute for 3 hours.

3) After digestion, 30 ml of Hanks' Balanced Salt Solution was added for dilution with agitation. The obtained liquid did not stratify, appearing slightly light-yellowish and gluey. The liquid was slowly filtered using a 100 $\mu$m cell strainer.

4) The filtrate was divided into 13 15ml centrifuge tubes, followed by centrifugation at 400 g and normal temperature for 6 minutes. After removal of supernatant, 1 ml of DMEM/F12 (commercially available from Gibco Company) was added into each tube to resuspend the cells, which were pooled into one tube and centrifuged at 400 g and normal temperature for 6 minutes. The supernatant was discarded and 1ml of DMEM/F12 added for resuspension to obtain the primary mesenchymal stem cells. The cells were counted and inoculated into a T25 culture flask comprising the IMedCell Culture Medium (commercially available from Shanghai IMedCell Biotechnology Co., Ltd) as the culture medium.

5) The medium was refreshed every 3 days. At day 9 of the cultivation, when cell density reaching 80% or more in the T25 culture flask on observation, the medium was removed and 1 mL of TrypLE digestion solution was added to the bottom of the flask, followed by digestion at 37 °C for 3 minutes in the incubator. 3 mL of the IMedCell Culture Medium was added into the culture flask using a 10 mL pipette to end digestion. The supernatant was collected into a 15 mL centrifuge tube, the bottom of the flask was rinsed with 1 mL of the IMedCell Culture Medium once and the rinse was pooled into the tube. After centrifugation at 1500 r.p.m. for 5 minutes, the supernatant was discarded and 1 mL of the IMedCell Culture Medium added for resuspension. The cells were inoculated into a T25 culture flask at a specified count and then cultured into the umbilical mesenchymal stem cells of passage P1.

6) The cells were passaged in series. The cells of P6 were inoculated at the density of 5000 cells/cm$^2$ into four T25 culture flasks, which were then cultured at 37 °C under the above-defined four conditions respectively. Four days later, the cells were harvested and suspected in the IMedCell Culture Medium and counted.

7) Calculation of colony-forming unit (CFU):

The cells cultured under different conditions were inoculated into separate 10 cm$^2$ petri dishes at 2200 cells/dish. After being evenly plated, the cells were respectively cultured under the same conditions as in step 6) in incubator. The cells were cultured for a period of 14 days, wherein the media were refreshed every 3 days and the growth profiles of the cell colonies were monitored.

When most of the colonies in the petri dishes had a cell count of over 50, each dish was added with 2 mL of 4% paraformaldehyde to fix the cells at 4 °C for 60 minutes. The cells were washed with PBS once. Each dish was added with 2 mL of clean and pure crystal violet staining solution and the cells were stained for 30 minutes.

The cells were washed, air-dried, photographed and counted for colonies. The colony-forming units (CFUs) were counted for each dish and the CFU rate calculated according to the formula: CFU rate =CFU number / the count of inoculation. The count of inoculation was 2200 in this experiment.

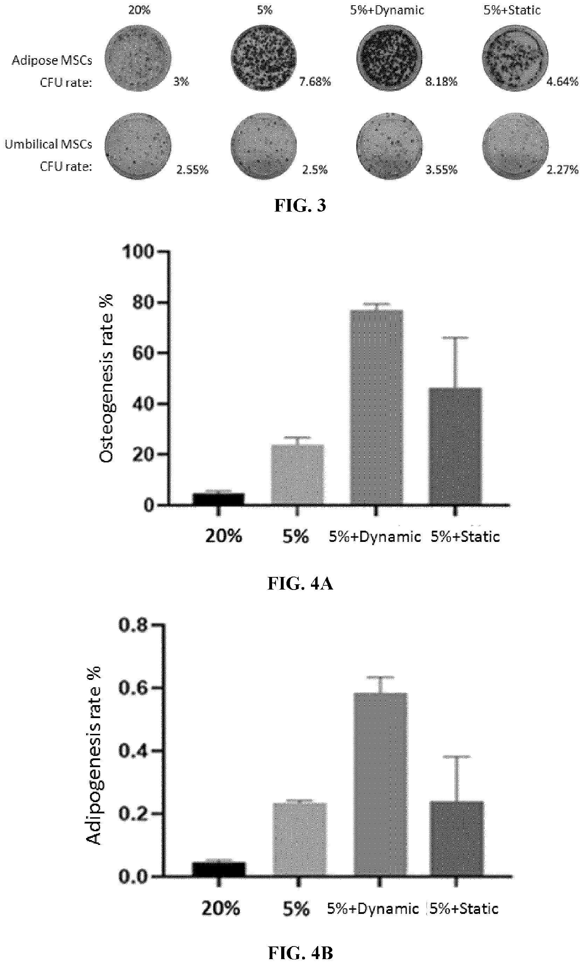

The results were shown in figure 3. The umbilical mesenchymal stem cells exhibited the highest CFU rate (as high as 3.55%) under the condition of hypoxia-and-dynamic pressure.

**Human adipose mesenchymal stem cell experiment:**

1) Intact surgery sample of human adipose tissue was obtained. The solid adipose tissue was washed with the cell wash solution, and the blood vessels and connective tissues visible to eyes were removed using a straight pointed ophthalmic scissor and a medical elbow dental ophthalmic forceps. 5 mL of fat was placed into a 50 mL centrifuge tube.

2) Two volumes (10 mL) of enzymolysis working solution (10mg/ mL of collagenase I in DMEM/F12 (as solvent)) was added into the tube, i.e., mixing at a ratio of 1 : 2. The adipose tissue was cut into 1 mm$^3$ paste with a sterilized medical straight narrow head fine scissors. The tubes with the cover tightened and being film-sealed were placed as tilted in the shaker at 37 °C for digestion at 80 r.p.m. for 1 hour till no significant fat particles. The digested adipose tissue was gently agitated via 4-5 blowings with a 10 mL pipette, into which 15 mL of wash solution was added and mixed by flipping the tube to end digestion, followed by centrifugation at 1500 r.p.m. and room temperature for 5 minutes and removal of the supernatant.

3) The pellet was resuspended with 20 mL of cell wash solution. All suspensions were each filtered through a 100 μm cell strainer, and the cell strainer was rinsed with 5 mL of cell wash solution. After centrifugation at 1500 r.p.m. and room temperature for 5 minutes, the supernatant was discarded and 5 mL of DMEM-F12 added to resuspend the cells. The cells were counted and inoculated into a T25 culture flask comprising the Amniocyte Culture Medium (commercially available from Guangzhou Baiyunshan Baidi Biopharmaceutical Co., Ltd.) as the culture medium. The cells were cultured at 37 °C for 48 hours, then the non-adherent erythrocytes were washed out by medium exchange and the culturing continued with medium being refreshed every 4 days to thereby obtain the primary mesenchymal stem cells. The cells were ready for passaging when reaching 80% - 90% confluence at day 7 to day 9 of the cultivation. TrypLE was added for digestion at room temperature for 2 minutes, then 2 volumes of the Amniocyte Culture Medium was added to end digestion. After centrifugation, the supernatant was discarded and 1 mL of the Amniocyte Culture Medium was added to resuspend the cells. The cells were inoculated into a T25 culture flask at a specified count and then cultured into the adipose mesenchymal stem cells of passage P1.

4) The cells were passaged in series. The cells of P4 were inoculated into four T25 culture flasks at the density of 5000 cells/cm$^2$, which were then cultured at 37 °C under the above-defined four conditions respectively. Four days later, the cells were harvested into the Amniocyte Culture Medium to form cell suspension and counted.

5) The colony-forming units (CFUs) were calculated as described above.

As shown by the results in figure 3, the adipose mesenchymal stem cells cultured under hypoxia exhibited significantly increased CFUs than those cultured under normoxia. The adipose mesenchymal stem cells exhibited the highest CFU rate (as high as 8.18%) under the condition of hypoxia-and-dynamic pressure.

**Example 3: Comparison of osteogenic differentiation and adipogenic differentiation between hair follicle mesenchymal stem cells cultured under different conditions**

Hair follicle mesenchymal stem cells were prepared and passaged according to Example 1. The cells were respectively maintained under the four conditions for culturing and passaging since the primary generation. The hair follicle mesenchymal stem cells of P5 were harvested for induced osteogenic differentiation and adipogenic differentiation.

**Osteogenic differentiation and staining:**

$2.5 \times 10^5$ cells as measured according to viable cell density were collected into a 15 mL centrifuge tube, into which the Complete Growth Medium was added to a total volume of 2.5 mL and a final concentration of $1.0 \times 10^5$ cells/mL. The cells (1 mL of culture per well, i.e., $1.0 \times 10^5$ cells/well) were cultured in incubators under the four conditions for 3 - 4 days until 90% - 100% confluence. On a clean bench, the medium in each well was aspirated and discarded, then 1 mL/well of the Osteogenesis-inducing Differentiation Complete Medium (commercially available from Cyagen Biotechnology Co., Ltd.) was added. The medium was refreshed every 3 days. At day 14 of the osteogenic differentiation, the cells were stained with Alizarin Red S for osteogenic differentiation. The resultant osteogenesis rates (%) were shown in figure 4A.

**Adipogenic differentiation and staining:**

$2.5 \times 10^5$ cells as measured according to viable cell density were collected into a 15 mL centrifuge tube, into which the Complete Growth Medium was added to a total volume of 2.5 mL and a final concentration of $1.0 \times 10^5$ cells/mL. The cells (1 mL of culture per well, i.e., a cell concentration of $1.0 \times 10^5$ cells/well) were cultured in incubators under the four conditions for 3 - 4 days till 90% - 100% confluence. On a clean bench, the medium in each well was aspirated and discarded, then 1 mL/well of the Adipogenesis-inducing Differentiation Medium (commercially available from STEMCELL) was added. The medium was refreshed every 3 days. At day 14 of the adipogenic differentiation, the cells were stained with Oil Red O for adipogenic differentiation. The resultant adipogenesis rates (%) were shown in figure 4B.
As shown by the results, the mesenchymal stem cells cultured under hypoxia exhibited enhanced potentials of osteogenic and adipogenic differentiation than those cultured under normoxia. Pressurization further enhanced the differentiation potentials. In particular, mesenchymal stem cells under the culturing condition of hypoxia-and-dynamic pressure exhibited significantly enhanced potentials of osteogenic and adipogenic differentiation.

**Example 4: Comparison of sternness and senescence gene expression between mesenchymal stem cells cultured under different conditions**

**Preparation and culturing of human hair follicle mesenchymal stem cells:**

Hair follicle mesenchymal stem cells were prepared and passaged according to Example 1, wherein the cells were respectively maintained under the four conditions for culturing and passaging since the primary generation, except that the Cyagen Culture Medium (commercially available from Cyagen (Guangzhou) Biotechnology Co., Ltd.) was used instead of the Amniocyte Culture Medium. Hair follicle mesenchymal stem cells of P3 and P5 were harvested and stored for assays of sternness gene expression.

**Preparation and culturing of human endometrial mesenchymal stem cells:**

1) Tissue separation: Menstrual blood in a 50 mL centrifuge tube was sequentially filtered through an 18-mesh stainless cell strainer, a 36-mesh stainless cell strainer, an 80-mesh stainless cell strainer and a 100 $\mu$m filter membrane.
2) Separation via the mononuclear method:
The menstrual blood filtrate obtained in step 1) was pre-centrifuged at 400g, 20 °C for 10 minutes. Most of the supernatant was removed, leaving 2 mL of the supernatant and the cell pellet at the bottom. The pellet was diluted and mixed with the wash solution, and the total volume of the diluted sample was 2 times of the volume of pure blood. 16 mL of the Lymphocyte Separation Medium at room temperature was fetched using a syringe and transferred into a 50 mL SepTube to completely fill the bottom chamber. 20 mL of the blood sample was directly added along the tube wall, followed by centrifugation at 800g, 20 °C for 15 minutes, whereby the sample stratified into four layers, wherein above the plate were the plasma layer and the buffy coat layer and below the plate were the Lymphocyte Separation Medium layer and the erythrocyte layer. Most part (around 15 mL) of the plasma layer on top was aspirated and discarded using a 3 mL of Pasteur Pipette. The remaining 2 mL of the plasma layer and the buffy coat was carefully pipetted into a 50 mL centrifuge tube using a 3 mL of Pasteur Pipette.

3) Tissue culturing

After washing with the wash solution of two volumes relative to the buffy coat, 10 mL of wash solution was added to resuspend the pellet in each tube. The resuspended pellets were pooled (40 mL/tube (the fraction in excess of 40 mL, if any, need be separated into one or more additional tubes) and centrifuged, with the supernatant being discarded. The pellet was washed again and the supernatant was discarded. 2ml of culture medium (7501 Culture Medium, commercially available from ScienCell Company) was added to resuspend the total cell pellet comprising the primary mesenchymal stem cells. The cells were counted and inoculated at the density of $3 \times 10^5$ cells/cm$^2$ into a 6-well plate, shaken to even and then cultured at 37 °C under the four conditions. The non-adherent erythrocytes were washed out by medium exchange 48 hours later and the culturing continued with medium being refreshed every 3 days. At day 7 of the cultivation, when cell density reached 80% or more in the 6-well plate on observation, the culture was subjected to tryptic digestion at 37 °C for 3 minutes. Then, 2 volumes of the 7501 Culture Medium was added to end digestion. After centrifugation, the supernatant was discarded and the cells were resuspended in 1 mL of the 7501 Culture Medium. The cells were inoculated into a T25 culture flask at a specified count and then cultured into the umbilical mesenchymal stem cells of passage P1.

The cells were passaged in series to P8 and maintained under the four conditions throughout the process respectively. Endometrial mesenchymal stem cells of P6 and P8 were harvested and stored for assays of senescence gene expression.

**Assays of sternness gene expression and senescence gene expression:**

**Total RNA Extraction:** The process included the following steps: collecting mesenchymal stem cells in a 1.5 mL centrifuge tube, washing the cells twice with PBS then adding 1 mL of RNAiso Plus (Lysis solution, commercially available from Takara) and leaving standing on ice; adding $200\mu L$ chloroform, mixing well and leaving standing on ice for 5mins, then centrifuging at 4 °C and 12000 r.p.m. for 15 minutes; gently pipetting $500\mu L$ of the transparent aqueous supernatant into a 1.5 mL centrifuge tube, adding an equal volume of isopropanol and mixing well by gently flipping the tube 8 times then leaving standing on ice for 15 minutes; centrifuging at 4 °C and 12000 r.p.m. for 15 minutes, when white pellet was observed at tube bottom; discarding the supernatant, washing the pellet with 75% ethanol, centrifuging again, discarding the supernatant and opening the tube cover to air-dry the pellet at room temperature; adding 15 - $20\mu L$ DNase/RNase Free H$_2$O (Invitrogen™) and mixing well to completely dissolving the pellet.

**Reverse transcription of total RNA into cDNA.** The TransScript One - Step gDNA Removal and cDNA Synthesis Super Mix kit (AT311-03, Beijing TransGen Biotech Co., Ltd.) was used for reverse transcription. Specifically, by following the manufacturer's instruction, $1\mu g$ total RNA was measured, into which Dnase/RNase Free H$_2$O was added to $7\mu L$, followed by addition of $1\mu L$ random primers, which was mixed at 65 °C for 5 minutes to obtain the RNA Mix. Then, the other components were added according to the manufacturer's instruction to obtain the reverse transcription system. The reverse transcription was conducted in a gradient PCR cycler according to the following protocol: 25 °C for 10 minutes, 42 °C for 30 minutes, and 85 °C for 5 seconds.

**Reverse transcription system:**

| Component | Volume |
|---|---|
| RNA Mix | 8 $\mu$L |
| 2×TS Reaction Mix | 10 $\mu$L |
| TransScript RT/RI Enzyme Mix | 1 $\mu$L |
| gDNA Remover | 1 $\mu$L |
| Total volume | 20 $\mu$L |

**qPCR for detection of gene expression.** The PerfectStart Green qPCR Super Mix kit (AQ601-04, Beijing TransGen Biotech Co., Ltd.) was used for qPCR. Specifically, 180μL of DNase/RNase Free Water (Invitrogen™) was added into 20μL of the reverse transcription system comprising the cDNA to obtain the cDNA template solution. By following the manufacturer's instruction, 20 μL/well of the reaction system according to the following table was added into a 96-well plate for fluorescence quantitative PCR. The plate was film-sealed and centrifuged at 4000 r.p.m. and 4 °C for 1 minute before PCR. The PCR parameters were as follows: 94 °C for 30 sec of denatuation followed by 40 cycles of 94 °C for 5 sec, 60 °C for 15 sec and 72 °C for 15 sec. Ct values were detected.

Reaction system:

| Component | Volume |
|---|---|
| cDNA template | 3 μL |
| Upstream primer (10μM) | 0.5 μL |
| Downstream primer (10μM) | 0.5 μL |
| 2×TransScript Top/Tip Green qPCR SurperMix | 10 μL |
| DNase/RNase-free distilled water | 6 μL |
| Total volume | 20 μL |

PCR primers:

| | | | |
|---|---|---|---|
| Internal Reference gene | GAPDH | Upstream primer | TTCGTCATGGGTGTGAACCA (SEQ ID NO: 1) |
| | | Downstream primer | CTGTGGTCATGAGTCCTTCCA (SEQ ID NO: 2) |
| Senescence genes | P53 | Upstream primer | GAGGTTGGCTCTGACTGTACC (SEQ ID NO: 3) |
| | | Downstream primer | TCCGTCCCAGTAGATTACCAC (SEQ ID NO: 4) |
| | P21 | Upstream primer | TCACTGTCTTGTACCCTTGTGC (SEQ ID NO: 5) |
| | | Downstream primer | GGATTAGGGCTTCCTCTTGG (SEQ ID NO: 6) |
| | P16 | Upstream primer | ATGGAGCCTTCGGCTGACT (SEQ ID NO: 7) |
| | | Downstream primer | GTAACTATTCGGTGCGTTGGG (SEQ ID NO: 8) |
| Sternness genes | Nanog | Upstream primer | GCATCCGACTGTAAAGAATCTTCA (SEQ ID NO: 9) |
| | | Downstream primer | CATCTCAGCAGAAGACATTTGCA (SEQ ID NO: 10) |
| | Sox2 | Upstream primer | GGGAATGGACCTTGTATAG (SEQ ID NO: 11) |
| | | Downstream primer | GCAAAGCTCCTACCGTACCA (SEQ ID NO: 12) |
| | Sall4 | Upstream primer | GCCGCACTGAGATGGAAG (SEQ ID NO: 13) |
| | | Downstream primer | AATGTCGAGGGTCCCACA (SEQ ID NO: 14) |
| | Klf4 | Upstream primer | GGGAGAAGACACTGCGTCA (SEQ ID NO: 15) |
| | | Downstream primer | GGAAGCACTGGGGGAAGT (SEQ ID NO: 16) |

**Data calculation and processing:**

The Ct value represents the number of PCR cycles for the fluorescence measurement of the amplification product to arrive at the set threshold, where the number of templates may be expressed as $M \times 2^{Ct}$ (M refers to the initial number of templates).

Assuming Mi and Cti are assigned to the target gene (i.e., a senescence gene or a sternness gene) while $M_2$

and $Ct_2$ are assigned to the internal reference gene (i.e., a gene of constant expression in mesenchymal stem cells, such as the GAPDH gene used in this example),

$$M_1 \times 2^{Ct1} = M_2 \times 2^{Ct2},$$

and

$$M_1/M_2 = 2^{Ct2}/2^{Ct1} = 2^{-(Ct1-Ct2)}.$$

Thereby, the measurement of $M_1/M_2$, i.e., $2^{-(Ct1-Ct2)}$ eliminates the influence from the differentia between cell amounts and accurately reflects the initial number of target gene templates for the same cell amount.

The measurements of sternness gene expression in human hair follicle mesenchymal stem cells were summarized in Table 1. As seen, for both P3 and P5, the hair follicle mesenchymal stem cells cultured under the condition of hypoxia-and-dynamic pressure exhibited the highest expression of the four sternness genes.

## Table 1: Expression of four stemness genes under different culturing conditions

| Expression of Stemness genes | | | | | | | | |
|---|---|---|---|---|---|---|---|---|
| Cell and passage | human hair follicle mesenchymal stem cells P3 | | | | human hair follicle mesenchymal stem cells P5 | | | |
| Culturing condition | 20% | 5% | 5%+Dynamic | 5%+Static | 20% | 5% | 5%+Dynamic | 5%+Static |
| Klf4 ($\times 10^2$) | 1.36 | 1.54 | **2.52** | 0.54 | 0.30 | 0.57 | **0.65** | 0.58 |
| Nanog ($\times 10^4$) | 3.58 | 1.98 | **7.10** | 1.23 | 2.08 | 1.49 | **10.28** | 1.33 |
| Sall4 ($\times 10^5$) | 1.04 | 4.24 | **11.45** | 2.29 | 1.57 | 5.43 | **8.12** | 4.03 |
| Sox2 ($\times 10^4$) | 4.36 | 5.74 | **7.96** | 2.13 | 0.61 | 1.38 | **3.96** | 0.58 |

The measurements of senescence gene expression in human endometrial mesenchymal stem cells were summarized in Table 2. As seen, for both P6 and P8, the endometrial mesenchymal stem cells cultured under the condition of hypoxia-and-dynamic pressure exhibited the lowest expression of the three senescence genes.

Table 2: Expression of three senescence genes under different culturing conditions

| Expression of senescence genes | | | | | | | | |
|---|---|---|---|---|---|---|---|---|
| Cell type | Human endometrial mesenchymal stem cells P6 | | | | Human endometrial mesenchymal stem cells P8 | | | |
| Culturing condition | 20% | 5% | 5%+Dynamic | 5%+Static | 20% | 5% | 5%+Dynamic | 5%+Static |
| P53 ($\times 10^2$) | 1.95 | 2.64 | **1.56** | 1.78 | 1.78 | 2.27 | **1.43** | 17.82 |
| P16 ($\times 10^4$) | 11.30 | 2.74 | **1.83** | 3.57 | 4.09 | 0.91 | **0.54** | 1.16 |
| P21 ($\times 10^2$) | 1.06 | 0.92 | **0.59** | 0.89 | 1.65 | 1.98 | **1.34** | 1.57 |

Summing up, the culturing condition of hypoxia-and-dynamic pressure promotes up-regulation of sternness genes and down-regulation of senescence genes in mesenchymal stem cells.

**Example 5: Comparison of doubling time and expansion fold between human hair follicle mesenchymal stem cells cultured under different conditions**

The T75 culture flask comprising the human hair follicle mesenchymal stem cells was taken out from the carbon dioxide incubator and the medium there in was discarded. 10 mL of PBS was added along the cell-free side, the flask was put right and gently shaken to wash the remaining culture medium, the PBS was then discarded and the wash was repeated twice. The culture flask comprising the cells was added with 3 mL of trypsin TrypLE then gently shaken to make the enzymolysis solution TryLE evenly spread along the bottom of the culture flask. The flask was then put into the incubator of 37 °C and 5% carbon dioxide for digestion of 1 - 2 minutes. The cells in the culture flask were examined under an inverted microscope and were observed with cytoplasm shrinking, cell gap increasing, rounding and increased lucency. The culture flask was shaken to detach the adherent cells from the bottom surface and then added with 6 mL of PBS to dilute the digestion enzyme solution. The cells were suspended by blowing to the bottom surface of the flask using a 10 mL pipette, without bubbling damaging the cells.

The suspension in the culture flask was all transferred into a 15 mL centrifuge tube. The bottom surface of the flask was rinsed with 6 mL of PBS and the rinse was pooled into the tube. The tube was centrifuged at room temperature (around 20 °C) and 400g for 5 minutes. The supernatant was removed using a vacuum suction pump without agitating the pellet. 3 mL of pre-warmed culture medium was added into the centrifuge tube using a 5 mL pipette. The cells were suspended and mixed via 15 blowings using a 100 - 1000 $\mu$L pipette. 11$\mu$L of the suspension was mixed with 11 $\mu$L of AO/PI staining solution and 20 $\mu$L of the mixture was added into a counting plate for counting in an automated cell counter to determine the amount of inoculation.

The culture flask was pre-filled with 15 mL of the complete culture medium. The volume of suspension for inoculation was calculated using the passaging density being 5000 cells/cm$^2$. The cell suspension was agitated via 10 blowings using a 100 - 1000 $\mu$L pipette, and then the suspension with well calculated volume was transferred into the T75 culture flask comprising the complete culture medium. The cells were mixed by shaking the flask in the way of drawing "X" or "8" and then cultured under the following three conditions in an incubator at 37 °C, 5% $CO_2$, with the flask standing straight: normoxia-and-normal pressure, hypoxia (3%)-and-normal pressure and hypoxia-and-static pressure (3%+Static), to thereby obtain hair follicle mesenchymal stem cells of P1.

The cells were passaged in series to P6 and maintained under the three conditions throughout the process respectively. For each passage, Expansion fold = cell count of the recovery/cell count of the inoculation. In this Example, when the passaging density was accurately 5000 cells/cm$^2$ and the surface area of a T75 culture flask being 75 cm$^2$, each inoculation into a T75 culture flask comprised 3.75$\times 10^5$ cells and the calculation of expansion fold was "expansion fold = cell count of each recovery / (3.75$\times 10^5$)". The doubling times under different conditions were as shown in Table 3 and figure 8. The expansion folds under different conditions were as shown in Table 4 and figure 9.

Table 3: Doubling times (hours) under different conditions

| Condition \ Passage | P2 | P3 | P4 | P5 | P6 |
|---|---|---|---|---|---|
| normoxia-and-normal pressure | 39.13 | 32.7 | 41.55 | 47 | 98.6 |
| hypoxia-and-static pressure | 37.09 | 29.72 | 34.1 | 37.71 | 91.52 |
| hypoxia-and-normal pressure | 39.13 | 31.01 | 42.73 | 49.61 | 104.8 |

Table 4: Expansion folds under different conditions

| Condition \ Passage | P2 | P3 | P4 | P5 | P6 |
|---|---|---|---|---|---|
| normoxia-and-normal pressure | 6.58 | 9.6 | 9.96 | 9.12 | 5.75 |
| hypoxia-and-static pressure | 9.84 | 15.36 | 21.04 | 15.84 | 10.98 |
| hypoxia-and-normal pressure | 9.58 | 15 | 12.75 | 9.82 | 6.59 |

As seen from Table 3 and figure 8, the human hair follicle mesenchymal stem cells cultured under hypoxia-and-static pressure exhibited decreased doubling time than those cultured under normoxia-and-normal pressure and those cultured under hypoxia-and-normal pressure. As seen from Table 4 and figure 9, the human hair follicle mesenchymal stem cells cultured under hypoxia-and-static pressure exhibited increased expansion fold than those cultured under normoxia-and-normal pressure and those cultured under hypoxia-and-normal pressure.

**Example 6: Comparison of osteogenic differentiation and chondrogenic differentiation between hair follicle mesenchymal stem cells cultured under different conditions**

Hair follicle mesenchymal stem cells were prepared and passaged according to Example 1, except that the cells were cultured under the following two conditions since the primary cells: normoxia (20%)-and-normal pressure and hypoxia-and-dynamic pressure (3%+Dynamic). Hair follicle mesenchymal stem cells of P4 and P5 were harvested for assays of osteogenic differentiation and chondrogenic differentiation.

**Osteogenic differentiation and staining:**

Hair follicle mesenchymal stem cells of P4 cultured under the two conditions of normoxia (20%)-and-normal pressure and hypoxia-and-dynamic pressure (3%+Dynamic) were induced for osteogenic differentiation and osteogenesis rates monitored according to the steps of osteogenic differentiation and staining in Example 3. The resultant osteogenesis rates (%) were shown in Table 5 below.

Table 5: Osteogenesis rates of hair follicle MSCs cultured under different conditions

| Culturing condition | Osteogenesis rate (%) |
|---|---|
| normoxia (20%)-and-normal pressure | 8.78 |
| hypoxia-and-dynamic pressure (3%+Dynamic) | 95.67 |

**Chondrogenic differentiation and staining:**

Hair follicle mesenchymal stem cells of P5 were resuspended in the Mesenchymal Stem Cells Complete Growth Medium at $1.0 \times 10^7$ cells/ml. The suspension was added evenly in drops onto the inner side of the cover of a $10 cm^2$ petri dish and cultured under the two conditions of normoxia (20%)-and-normal pressure and hypoxia-and-dynamic pressure (3%+Dynamic) at 37 °C for 24 hours. On a clean bench, spherical drops were pipetted into 0.5 mL of the Chondrogenic Differentiation Medium (commercially available from STEMCELL) in a low-adsorption 24-well plate at 10 spheres per well and at least 3 wells per group for continued culturing under the two conditions. The medium was refreshed every 3 days. At day 28 of the chondrogenic differentiation, cell microspheres were collected and stained with Alcian blue. The result was shown in figure 10.

As seen from Table 5, hair follicle mesenchymal stem cells under the condition of hypoxia-and-dynamic pressure exhibited enhanced potential of chondrogenic differentiation than those under the condition of normoxia-and-normal pressure. As seen from figure 10, for hair follicle mesenchymal stem cells cultured under both conditions, i.e., normoxia-and-normal pressure and hypoxia-and-dynamic pressure, apparent blue (dark) zones in tissue were observed, which indicated the capacity of chondrogenesis. And, the hair follicle mesenchymal stem cells cultured under hypoxia-and-dynamic pressure exhibited enhanced potential of chondrogenic differentiation than those cultured under normoxia-and-normal pressure.

Despite of the described with specific examples of the invention, it will be apparent to those skilled in the art that various changes and modifications can be made in the present invention without departing from the spirit and scope of the invention, which all fall within the scope of the invention as defined by the appended claims.

**Claims**

1. A method of culturing stem cells, **characterized in that** said method comprises a step of culturing stem cells in an atmospheric environment comprising an additional pressure applied in addition to an atmospheric pressure, wherein the additional pressure is 60 - 140 mmHg, such as 70 - 120 mmHg, 75 - 115 mmHg, 80 - 110 mmHg, 85 - 100 mmHg or 90 - 95 mmHg.

2. The method of claim 1, **characterized in that** said additional pressure changes by way of periodic fluctuation within the range of 60 - 140 mmHg, such as 70 - 120 mmHg or 75 - 115 mmHg, wherein said fluctuation is preferably a sinusoidal or sinusoid-like periodic fluctuation.

3. The method of claim 2, **characterized in that** said periodic fluctuation has a frequency of 12 - 100 times/minute, such as 13 - 18 times/minute, 40 - 80 times/minute, 13 - 15 times/minute or 50 - 70 times/minute.

4. The method of claim 1, **characterized in that** said additional pressure is constant.

5. The method of claim 1, **characterized in that** said atmospheric environment comprises an oxygen concentration of 2% - 20%, such as 2% - 8%, 3% - 7%, 4% - 7% or 5% - 7%.

6. The method of claim 1, **characterized in that** the stem cells in said step are primary stem cells and/or passaged stem cells.

7. The method of claim 1, **characterized in that** said stem cells are human stem cells.

8. The method of claim 1, **characterized in that** said stem cells are mesenchymal stem cells, such as human adipose mesenchymal stem cells, human endometrial mesenchymal stem cells, human hair follicle mesenchymal stem cells and human umbilical mesenchymal stem cells.

9. The method of claim 1, **characterized in that** said culturing is conducted at a temperature of 36.5 - 37.5 °C, preferably 37 °C.

10. A stem cell obtained according to the method of any one of claims 1-9.

**FIG. 1**

**FIG. 2**

**FIG. 3**

**FIG. 4A**

**FIG. 4B**

**FIG. 5**

**FIG. 6**

**FIG. 7**

**FIG. 8**

## Expansion Fold

Legend:
- Normoxia & normal pressure
- Hypoxia & static pressure
- Hypoxia & normal pressure

**FIG. 9**

Normoxia-and normal pressure

Hypoxia-and-dynamic pressure

**FIG. 10**

## INTERNATIONAL SEARCH REPORT

| International application No. |
|---|
| **PCT/CN2021/106399** |

**A.    CLASSIFICATION OF SUBJECT MATTER**

C12N 5/0775(2010.01)i

According to International Patent Classification (IPC) or to both national classification and IPC

**B.    FIELDS SEARCHED**

Minimum documentation searched (classification system followed by classification symbols)

C12N

Documentation searched other than minimum documentation to the extent that such documents are included in the fields searched

Electronic data base consulted during the international search (name of data base and, where practicable, search terms used)

DWPI, SIPOABS, CNABS, USTXT, JPTXT, EPTXT, WOTXT, CNTXT, CNKI, WEB OF SCIENCE, PUBMED, 万方数据库: 干细胞, 人间充质干细胞, 培养, 额外压力, 波动, stem cell, human mesenchymal stem cell, culture, extra pressure, wave.

**C.    DOCUMENTS CONSIDERED TO BE RELEVANT**

| Category* | Citation of document, with indication, where appropriate, of the relevant passages | Relevant to claim No. |
|---|---|---|
| X | CN 107119011 A (LIU, Liwei) 01 September 2017 (2017-09-01)<br>      see abstract | 10 |
| A | WO 2012031263 A2 (LIFE TECHNOLOGIES CORP. et al.) 08 March 2012 (2012-03-08)<br>      see entire document | 1-10 |
| X | 彭磊 等 (PENG, Lei et al.). "持续性压力培养环境对兔骨髓基质干细胞增殖的影响 (Effect of continuous Pressure on the Proliferation of Rabbit's Bone Marrow Stem Cells)" 中国组织工程研究与临床康复 (Journal of Clinical Rehabilitative Tissue Engineering Research), Vol. 13, No. 27, 02 July 2009 (2009-07-02),<br>ISSN: 1673-8225,<br>      pages 5239-5242, see abstract | 1, 4-9 |
| Y | 彭磊 等 (PENG, Lei et al.). "持续性压力培养环境对兔骨髓基质干细胞增殖的影响 (Effect of continuous Pressure on the Proliferation of Rabbit's Bone Marrow Stem Cells)" 中国组织工程研究与临床康复 (Journal of Clinical Rehabilitative Tissue Engineering Research), Vol. 13, No. 27, 02 July 2009 (2009-07-02),<br>ISSN: 1673-8225,<br>      pages 5239-5242, see abstract | 2, 3 |

☑ Further documents are listed in the continuation of Box C.        ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **10 September 2021** | **28 September 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT

| | International application No. |
|---|---|
| | **PCT/CN2021/106399** |

### C.    DOCUMENTS CONSIDERED TO BE RELEVANT

| Category* | Citation of document, with indication, where appropriate, of the relevant passages | Relevant to claim No. |
|---|---|---|
| Y | 梁玲玲 等 (LIANG, Lingling et al.). "压力仿生培养对兔角膜内皮细胞调控的研究 (Research on Regulation of Rabbit Corneal Endothelial Cells with Pressure Bionic Cultivation)" <br> 眼科 (Ophthalmology in China), Vol. 26, No. 1, 25 January 2017 (2017-01-25), ISSN: 1004-4469, <br>       pages 56-60, see abstract | 2, 3 |

Form PCT/ISA/210 (second sheet) (January 2015)

**INTERNATIONAL SEARCH REPORT**

International application No.

**PCT/CN2021/106399**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

<div align="center">

**INTERNATIONAL SEARCH REPORT**
**Information on patent family members**

</div>

International application No.

**PCT/CN2021/106399**

| Patent document cited in search report | | | Publication date (day/month/year) | Patent family member(s) | | | Publication date (day/month/year) |
|---|---|---|---|---|---|---|---|
| CN | 107119011 | A | 01 September 2017 | None | | | |
| WO | 2012031263 | A2 | 08 March 2012 | CN | 105112361 | B | 16 November 2018 |
| | | | | CN | 103153318 | A | 12 June 2013 |
| | | | | CN | 105112361 | A | 02 December 2015 |
| | | | | CN | 103153318 | B | 02 September 2015 |
| | | | | US | 2013164269 | A1 | 27 June 2013 |
| | | | | EP | 2611452 | A2 | 10 July 2013 |
| | | | | WO | 2012031263 | A3 | 03 May 2012 |
| | | | | EP | 2611452 | B1 | 24 October 2018 |

Form PCT/ISA/210 (patent family annex) (January 2015)